# EUROPEAN PATENT APPLICATION

(11) **EP 3 427 657 A1**
(43) Date of publication of application: **16.01.2019**
(21) Application number: 17181393.4
(22) Date of filing: 14.07.2017
(51) Int. Cl.: A61B 5/024

(54) **PHOTOPLETHYSMOGRAPHIC SENSOR AND METHOD OF PRODUCING A PHOTOPLETHYSMOGRAPHIC SENSOR**

(71) Applicant: Koninklijke Philips N.V., 5656 AE Eindhoven (NL)
(72) Inventor: Presura, Cristian Nicolae, 5656 AE Eindhoven (NL); Jacobi, Michel, 5656 AE Eindhoven (NL); Wismans, Antonius Johannes Joseph, 5656 AE Eindhoven (NL)
(74) Representative: de Haan, Poul Erik

(57) **Abstract**

A vital signs sensor is provided which has a PPG sensor including a light source (110) and a photo detector (120). The vital signs sensor also comprises a contact surface (101) which has a transparent portion (150) with a first protrusion (152) over the light unit and a second protrusion (153) over the photo detector as well as a gap (154) between the first and second protrusion (152, 153).

## Description

### FIELD OF THE INVENTION

The invention relates to an optical vital signs sensor as well as to a method of producing a vital signs sensor.

### BACKGROUND OF THE INVENTION

Optical heart rate sensors are well known to monitor or detect vital signs like a heart rate of a user. Such a heart rate sensor can be based on a photoplethysmographic (PPG) sensor and can be used to acquire a volumetric organ measurement. By means of optical pulse sensors or pulse oximeters, changes in light absorption of a human skin are detected and based on these measurements a heart rate or other vital signs of a user can be determined. The PPG sensors comprise a light source like a light emitting diode (LED) which is emitting light into the skin of a user. The emitted light is scattered in the skin and is at least partially absorbed by the blood. Part of the light exits the skin and can be captured by a photo detector. The amount of light that is captured by the photo detector can be an indication of the blood volume inside the skin of a user. A PPG sensor can thus monitor the perfusion of blood in the dermis and subcutaneous tissue of the skin through an absorption measurement at a specific wave length. If the blood volume is changed due to the pulsating heart, the scattered light coming back from the skin of the user is also changing. Therefore, by monitoring the detected light signal by means of the photo detector, a pulse of a user in his skin and thus the heart rate can be determined. Furthermore, compounds of the blood like oxygenated or de-oxygenated hemoglobin as well as oxygen saturation can be determined, when at least two colors are used.

The pulse signal of a heart beat can be detected by photoplethysmography PPG which is measuring a variation in the blood volume of the human tissue. In a PPG sensor, light emitting diodes e.g. at wavelengths between 520 nm (green) and 850 nm (infrared) are used to emit light onto the skin of a user. Transmission type PPG measurements are performed with light at wavelength ranges of 650 - 850 nm while reflective type PPG sensing is used at 520 - 570 nm.

Light is scattered in the skin of the user and some of the light is absorbed by blood. The reflected light exits the skin and can be detected by a photo diode. The output signal of the photo diode can therefore be an indication of the blood volume as well as the variation of the blood volume, i.e. the pulse in the skin of a user.

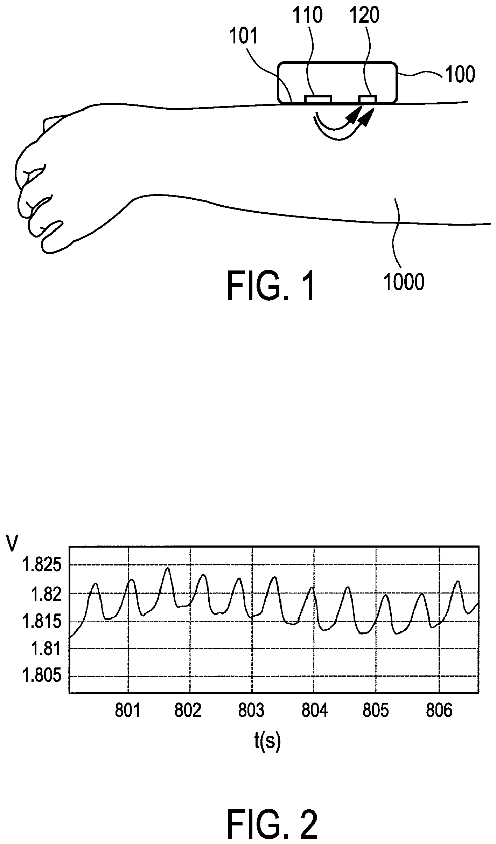

Fig. 1 shows a basic representation of an operational principle of a vital signs sensor. In Fig. 1, a heart rate sensor 100 with its contact surface 101 is arranged or placed on for example an arm or wrist of a user. The vital signs sensor 100 can be based on a photoplethysmograph PPG sensor. The contact surface 101 can be directly placed onto the skin 1000 of the user. The heart rate sensor 100 comprises at least one light source 110 and at least one photo detector 120. The light source 110 emits light e.g. via the contact surface 101 onto or in the skin 1000 of a user. Some of the light is reflected and the reflected light can be detected by the photo detector 120. Based on the reflected light, vital signs of a user like a heart rate can be determined.

Fig. 2 shows a graph indicating an output signal of a photo detector of the optical vital signs sensor of Fig. 1. In Fig. 2, the time t₍ₛ₎ as well as the output voltage V of the photo detector 120 is depicted. As can be seen, the peaks in the output voltage V can correspond to the pulse of the user and thereby the heart rate of the user can be determined. Accordingly, the photo detector 120 directly measures the pulse in the skin and the heart rate can be determined. This can be performed by counting the number of pulses per second.

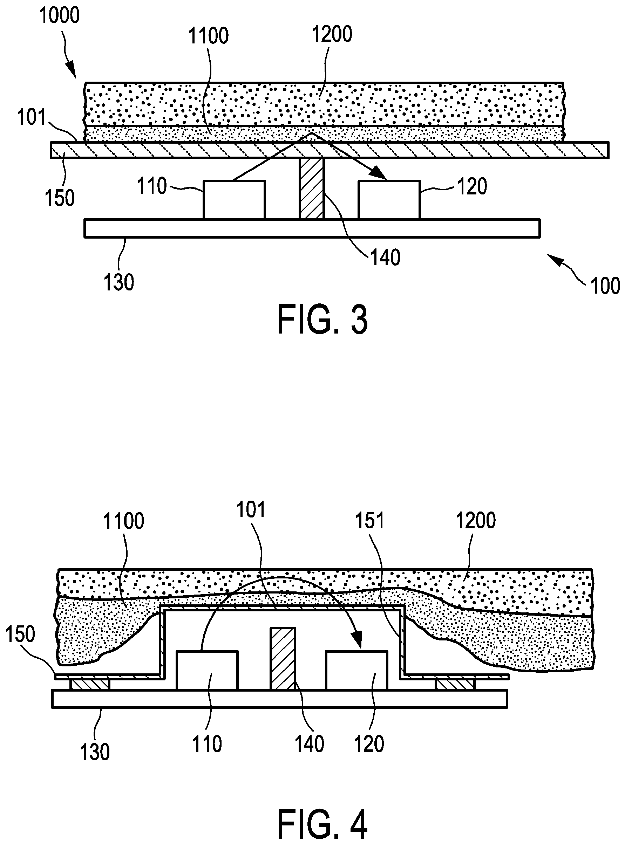

Fig. 3 shows a schematic cross section of a PPG sensor according to the prior art. The at least one light source 110 as well as the photo detector 120 can be arranged on a printed circuit board 130. Between the light unit 110 and the photo detector 120 a wall 140 can be provided. The contact surface 101 can be implemented as a transparent glass layer 150. The contact surface 110 can be placed against a skin 1000 of a user. The skin comprises a layer 1100 without pulsing blood and a deeper skin layer 1200 with pulsating blood.

Fig. 4 shows a further schematic cross section of a PPG sensor according to the prior art. The at least one light unit 110 and the photo detector 120 can be placed on a printed circuit board 130. The transparent glass layer 150 of the contact surface 101 has a protrusion 151 under which the light unit 110, the wall 130 and the detector 120 are arranged.

US 8,954,135 B2 also discloses a vital signs sensor having a printed circuit board with a light source and a photo detector. The light source and the photo detector are arranged in a protrusion covered by a protective transparent layer.

As can be seen from Fig. 3, the light from the light unit passes through the transparent glass 150 and the contact surface 101 and is reflected in the skin layer 1100 without pulsating blood. In contrast, the PPG sensor of Fig. 4 allows a transmission of the light from the light unit 110 through the layer without pulsating blood 1100 to the deeper skin layer 1200 with pulsating blood. Thus, the output signal of the photo detector 120 has an improved signal quality as compared to the PPG sensor of Fig. 3.

In the PPG sensor according to Fig. 3, the entire skin of the user is compressed when the PPG sensor 100 is pressed against the skin 1000 of the user (in particular the layer 1100 is compressed while the deeper skin layer is not equally compressed). As the light from the light unit 100 substantially only reaches the skin layer 1100 without pulsating blood, the signal quality of the photo detector will be deteriorated.

In the PPG sensor of Fig. 4, the contact surface is not planar but has a protrusion 151. Here, only the skin 1100 in the layer without pulsating blood is compressed, the light from the light unit 110 can reach into the deeper skin layer 1200 where the blood is pulsating. Thus, the signal quality of the photo detector 120 is improved. By the protrusion 151 of the glass 150, an amount of non pulsating blood is pushed away such that the light from the light unit 110 can reach into the deeper skin layers 1200.

### SUMMARY OF THE INVENTION

It is an object of the invention to provide a vital signs sensor with an improved signal quality.

According to an aspect of the invention, an optical vital signs sensor is provided which is able to measure or determine vital signs of a user. The optical vital signs sensor is implemented as photoplethysmographic sensor PPG. The vital signs sensor comprises at least one light source configured to generate light which is directed towards a skin of the user, at least one photo detector unit configured to detect light which is indicative of a reflection and/or transmission of light emitted in or from the skin of the user and a contact surface configured to be pressed against a skin of a user. The contact surface comprises a transparent portion having a first protrusion arranged over the at least one light unit, at least one second protrusion arranged over the at least one photo detector unit and a gap arranged between the first and second protrusion. With the gap between the first and second protrusion, it is possible to compress the skin of the user in the area of the protrusions while the skin in the gap between the two protrusions is not compressed as much. Thus, the light from the light unit can enter deeper into the skin of the user and can interact with the deeper skin layers where blood is pulsating. Thus, this leads to a significantly improved signal quality.

According to an aspect of the invention, the gap has a bottom with a width and side walls with a height and the width of the gap is proportional to the height of the side walls.

According to a further aspect of the invention, the bottom is flat and the side walls are perpendicular with respect to the bottom. This allows an easy fabrication of the gap between the first and second protrusion.

According to a further aspect of the invention, wherein a transition between the bottom and at one of the side walls of the gap and/or a transition between at least one of the side walls and one of the first and second protrusions is rounded and/or chamfered.

According to a further aspect of the invention, the contact surface comprises an elevated portion e.g. a plateau and the transparent portion with the protrusion is arranged at the elevated portion.

According to a further aspect of the invention, the at least one light unit and the photo detector unit are angled with respect to a printed circuit board.

According to a further aspect of the invention, the gap comprises side walls which are arranged at the same angle as the light unit and the photo detector.

According to a further aspect of the invention, the transparent portion comprises a transparent glass or a transparent plastic layer.

According to a further aspect of the invention, the transparent portion is transparent for at least a wavelength of the at least one light source.

According to a further aspect of the invention, the transparent portion is only transparent at the first and second protrusion but not at the other parts of the contact surface.

According to a further aspect of the invention, the transparent portion is only transparent at side walls of the gap, wherein the side walls are inclined with respect to a bottom of the gap.

According to an aspect of the invention, a method of producing an optical vital signs sensor is provided. The optical vital signs sensor is a PPG sensor. At least one light source is provided to generate light which is directed towards a skin of a user. At least one photo detector unit is provided to detect light which is indicative of a reflection or transmission of light emitted in or from the skin of the user. A contact surface is provided which can be pressed against the skin of the user. A transparent portion comprises at least one first protrusion arranged over the at least one light unit, at least one second protrusion arranged over the photo detector and a gap between the first and second protrusion.

According to an aspect of the invention, the vital signs sensor comprises a LED based PPG sensor. The LED light penetrates the skin of the user, is reflected and some of it can reach a photo detector. The output of the photo detector can be used to monitor a blood volume fraction and blood compounds like oxygenated and de-oxygenated hemoglobin. In particular, the amount of absorption or reflectance of the light from the LED light source can be used to determine the heart rate as well as the blood volume fraction or blood compounds. Furthermore, the PPG sensor according to the invention is therefore an optical sensor allowing a non-invasive measurement of vital signs of a user.

It shall be understood that a preferred embodiment of the present invention can also be a combination of the dependent claims or above embodiments or aspects with respective independent claims.

These and other aspects of the invention will be apparent from and elucidated with reference to the embodiment(s) described hereinafter.

### BRIEF DESCRIPTION OF THE DRAWINGS

In the following drawings:
Fig. 1 shows a basic representation of an operational principle of a vital signs sensor according to an aspect of the invention,
Fig. 2 shows a graph indicating an output signal of a photo detector of a PPG sensor according to the prior art,
Fig. 3 shows a schematic cross section of a PPG sensor according to the prior art,
Fig. 4 shows a schematic cross section of a PPG sensor according to the prior art,
Fig. 5 shows a cross section of an optical vital signs sensor according to an aspect of the invention,
Fig. 6 shows a schematic cross section of an optical vital signs sensor according to an aspect of the invention,
Fig. 7 shows a perspective cross section of an optical vital signs sensor according to an aspect of the invention, and
Fig. 8 shows a cross section of an optical vital signs sensor according to an aspect of the invention.

### DETAILED DESCRIPTION OF THE EMBODIMENTS

The optical vital signs sensor according to the invention is based on the optical vital signs sensor as described in Fig. 1 and can be implemented as a photoplethysmograph PPG sensor. The sensor 100 comprises a contact surface 101 which is placed directly onto the skin 1000 of the user. The PPG sensor 100 comprises at least one light source 110 and at least one photo detector 120. The light source emits light via a contact surface 101 onto or in the skin 1000 of a user. Some of the light is reflected and the reflected light can be detected by the photo detector 120. Based on the reflected light, vital signs of a user like the heart rate can be determined.

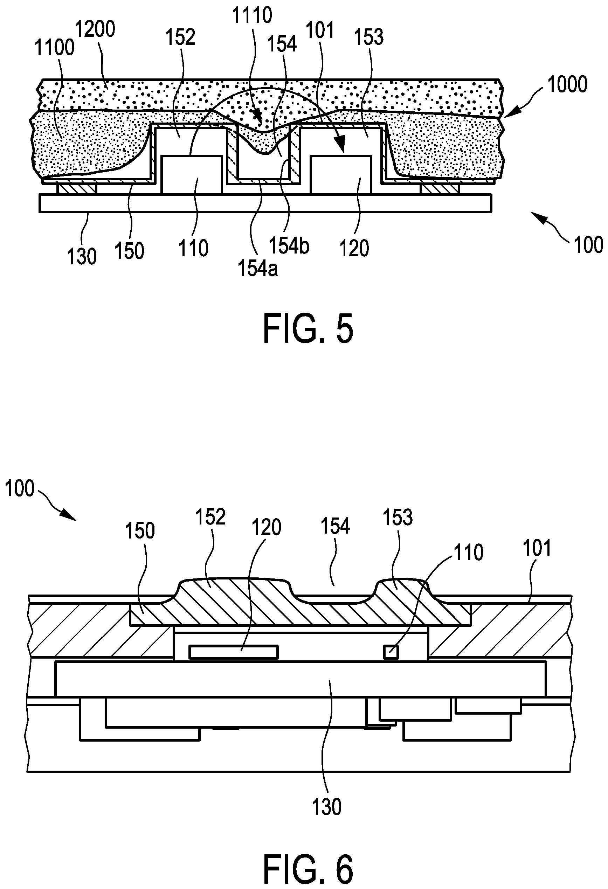

Fig. 5 shows a cross section of an optical vital signs sensor according to an aspect of the invention. The PPG sensor 100 comprises at least one light source 110, at least one photo detector 120 and a contact surface 101. The at least one light unit 110 emits light through the contact surface 101. The light is reflected in the skin 1000 of the user and can be detected by the photo detector 120. The light unit 110 which can be implemented as a light emitting diode LED and the photo detector 120 which can be implemented as a photo diode can be arranged on a printed circuit board 130.

The contact surface 101 of the PPG sensor can be implemented as or comprise a transparent portion 150.

According to an aspect of the invention, the contact surface 101 comprises a first protrusion 152 and a second protrusion 153 of the transparent portion 150. The first protrusion 152 is arranged over the at least one light unit 110 and the second protrusion 153 is arranged over the photo detector 120. Between the first and second protrusion 152, 153, a gap 154 of the contact surface 101 implemented by the transparent portion 150 is provided. In the gap 154, a skin fold 1110 can be present as the contact surface 110 is placed against the skin 1000 of a user. The protrusions 152, 153, are pressed deeper into the skin 1000 of a user such that part of the skin, namely the skin fold 1110 is present in the gap 154. This has the effect that substantially the skin at the first and second protrusions are compressed more than the skin in the area of the gap 154. Thus, the skin in the area of the gap 154 is less compressed as the skin in the area of the protrusions.

The gap 154 constitutes a regress surface which can have a bottom 154a with a width and side walls 154b with a height. According to an aspect of the invention, the width of the bottom 154a may be the same as the height of the side walls 154b. Alternatively, the width of the bottom 154a can be proportional to the height of the side walls 154b. Optionally, the gap can have a straight or flat bottom and side walls perpendicular with respect to the bottom.

The arrangement of the contact surface 101 according to an aspect of the invention is advantageous as it will allow an improved light penetration as non pulsating blood above the light unit 110 and the photo detector 120 can be pushed away. Because of the regress surface in form of the gap 154, part of the skin, namely a skin fold 1110, will be present in the gap between the first and second protrusion 152, 153 as can be seen in Fig. 5, the deeper skin layer 1200 with the pulsating blood will be closer to the light unit 110 and the photo detector 120 such that the signal quality of the photo detector 120 can be significantly improved. In particular, an improved modulation between the AC components and the DC components of the output signal of the photo detector can be achieved. Moreover, the PPG sensor can also be operated in an at least partial transmission mode which can allow a higher modulation. In general, a (partial) transmission mode allows for a better amplitude of the pulse, as the transmitted light travels throughout the blood vessel, while the reflected light travels only through a part of it.

Optionally, the sides of the protrusions may be blackened. The dimensions of the regress or gap 154 are important as the folding of the skin may not be sufficient if the height 154b is too small. On the other hand, if the height 154b is too high, then an optical short cut may be present.

According to an aspect of the invention, the width 154a of the gap can be between 0,3 - 0,9mm and is preferably 0,6mm.

The transparent portion 150 can be implemented as a transparent glass layer or a transparent plastic layer. In particular, the transparent parts of the transparent portions are designed such that they are transparent only for those wavelengths which correspond to the wavelength of the light sources. Optionally, only the portions of the first and second protrusions are transparent while the other parts may be non-transparent or semi-transparent.

Fig. 6 shows a schematic cross section of an optical vital signs sensor according to an aspect of the invention. The PPG sensor 100 according to Fig. 6 can be based on the PPG sensor according to Fig. 5. Thus, the PPG sensor 100 comprises at least one light unit 110 and at least one photo detector 120. The light unit 110 and the photo detector 120 can be placed on a printed circuit board 130. In the area of the photo detector and the light unit 120, a window with a glass layer 150 may be provided. The glass layer 150 may comprise a first and second protrusion 152, 153 with a gap 154 there between.

In comparison to the aspect of the invention illustrated in Fig. 5, the aspect illustrated in Fig. 6 is provided with a different geometry in terms of the shape of the first and second protrusions 152, 153 and the gap 154.

Rather than having side walls completely extending in a direction perpendicular to the bottom, the cross-section of the gap is similar to that of a bowl or tub, in having a rounded transition from the bottom to the side walls (in an alternative instead of a rounding or in combination with the rounding, there might be also a chamfered transition, while there transition may also be formed of multiple partial transitions). Furthermore, also the transition between the side walls and the surrounding first and second protrusion 152, 153 is provided in a rounded way. In the illustrated aspect, the radii of the roundings between the side walls and the top portions of the first and second protrusions 152, 153 are approximately equal. The invention, however, is not limited to this. Furthermore, also the radii of the roundings between the bottom and the side walls of the gap 154 are approximately equal and larger than the radii of the roundings between the side walls and the top portions of the first and second protrusions 152, 153. Again, the invention is not limited to this. Furthermore, the radius of a rounding does not have to be constant.

A particular benefit of the provision of rounded or chamfered transitions between the side walls of the gap 154 and the top portions of the first and second protrusions 152, 153 is an increased comfort to the user, in comparison to a transition in the form of a right angle, which might invoke a feeling similar to that of an edge of a knife.

An advantage in terms of the shape of the bottom to side wall transition is that the rounding (or chamfering) may allow for an easier cleaning.

Between the first and second protrusions 152, 153, i.e. at the location of the gap 154, there is provided a reduction of the pressure exerted on the skin, such that the skin may at least partially follow the shape of the glass layer 150, possibly all the way to the bottom of the gap 154. The resulting skin fold allows locally for a better blood flow by lower pressure and increases the penetration depth in the skin for the difference in depth level of the elements, 152,153 and 154.

In Fig. 6, the first and second protrusion 152, 153 are provided with different mechanical dimensions, which are adapted for the used optical components, e.g. as to the area used for the photo detector and the light source.

According to an aspect of the invention, the window may have an area of about 2mm x 2mm. The distance between the photo detector 120 and the light unit 110 can be for example 3,6mm.

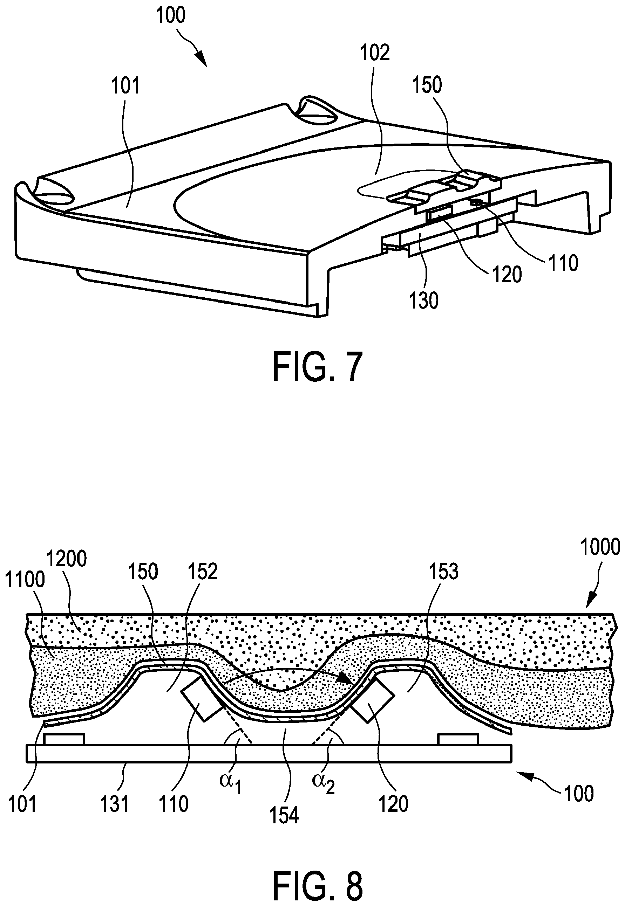

Fig. 7 shows a perspective cross section of an optical vital signs sensor according to an aspect of the invention. The optical vital signs sensor 100 according to Fig. 7 can be based on the optical vital signs sensor according to Fig. 6. In addition, the contact surface 101 of the optical vital signs sensor 100 may have an elevated portion 102 e.g. in form of a plateau with a small height. The window with the transparent portion 150 can be arranged in the plateau. This has the advantage that the pressure with which the PPG sensor is pressed against the skin of the user can be increased.

Optionally, the height of the elevated portion plateau 102 compared to the contact surface 101 can be about 0,8 mm.

According to a further aspect of the invention, the PPG sensor can be implemented as a wrist worn device like a smart watch and the PPG sensor can be oriented across the wrist of a user.

With such an optical vital signs sensor, the reflectivity can be increased by 30% and the modulation (signal quality) can be improved by x 1,3 and in particular by x 1,5 in the standing position.

Fig. 8 shows a cross section of an optical vital signs sensor according to an aspect of the invention. The PPG sensor 100 according to an aspect of the invention comprises at least one light unit 110, at least one photo detector 120 and a contact surface 101. The light unit 110 and the photo detector 120 can be coupled to a printed circuit board 130 which can be implemented as a flat circuit board 130 having a first direction or plane 131. The contact surface 110 of the PPG sensor 100 can be at least partly implemented by a transparent portion 150. The transparent portion 150 can have at least one first protrusion 152 and at least one second protrusion 153. The at least one first protrusion 152 is arranged at the at least one light unit 110 and the second protrusion 153 is arranged at the at least one photo detector 120. In other words, the at least one first protrusion 152 and the at least one second protrusion 153 are arranged over the at least one light unit 110 and the at least one photo detector 120, respectively.

According to an aspect of the invention, the at least one light unit 110 and the at least one photo detector 120 are arranged at a first and second angle α1, α2, respectively with respect to the printed circuit board 130, namely in particular with respect to the direction 131 of the printed circuit board. The two angles α1, α2 can have the same value.

According to an aspect of the invention, the gap can have a bottom and inclined side walls. The angle of the sidewalls may correspond to the angles α1, α2 of the light unit 110 and the photo detector 120.

As can be seen from Fig. 8, the light which is received by the photo detector 120 may correspond to light from the light unit 110 which has travelled through the layers 1100 and 1200. Thus, the PPG sensor may operate at least partially in the transmission mode.

The output signal of the PPG sensor gives an indication on the blood movement in vessels of a user. The quality of the output signal of the PPG sensor can depend on the blood flow rate, skin morphology and skin temperature. In addition, optical losses in the PPG sensor may also have an influence on the quality of the output signal of the PPG sensor. The optical efficiency of the PPG sensor can depend on reflection losses when light penetrates from one media into another. Furthermore, scattering of light at the surface of the skin of the user may also have an influence on the optical efficiency of the PPG sensor.

The PPG sensor or optical vital signs sensor according to an aspect of the invention can be implemented as a wrist device (like a watch or smart watch). The optical vital signs sensor can also be implemented as a device worn behind the ear of a user, e.g. like a hearing aid.

According to an aspect of the invention, the walls of the gap 154 can be inclined. Alternatively, the gap 154 can have side walls with a rounded slope.

According to a further aspect of the invention, the gap width is proportional to the height of the gap or the protrusions 152, 153. The gap width may depend on the surface of the photo detector.

According to a further aspect of the invention, the top of the first and second protrusion 152, 153 may be inclined or arranged at an angle with respect to the printed circuit board.

Other variations of the disclosed embodiment can be understood and effected by those skilled in the art in practicing the claimed invention from a study of the drawings, the disclosure and the appended claims.

In the claims, the word "comprising" does not exclude other elements or steps and in the indefinite article "a" or "an" does not exclude a plurality.

A single unit or device may fulfil the functions of several items recited in the claims. The mere fact that certain measures are recited in mutual different dependent claims does not indicate that a combination of these measurements cannot be used to advantage. A computer program may be stored/distributed on a suitable medium such as an optical storage medium or a solid state medium, supplied together with or as a part of other hardware, but may also be distributed in other forms such as via the internet or other wired or wireless telecommunication systems.

Any reference signs in the claims should not be construed as limiting the scope.

## Claims

1. Optical vital signs sensor (100) configured to measure or determine vital signs of a user, said optical vital signs sensor being a photoplethysmographic sensor (PPG), comprising
at least one light source (110) configured to generate light which is directed towards a skin (1000) of the user,
at least one photo detector unit (120) configured to detect light, wherein said light being indicative of a reflection and/or transmission of light emitted in or from the skin (1000) of a user, and
a contact surface (101) configured to be pressed against the skin (1000) of a user,
wherein the contact surface (101) comprises a transparent portion (150) with at least one first protrusion (152) arranged over the at least one light unit (110), at least one second protrusion (153) arranged over the at least one photo detector unit (120) and a gap (154) arranged between the first and second protrusion (152, 153).

2. Optical vital signs sensor (100) according to claim 1, wherein
the gap (154) has a bottom (154a) with a width and side walls (154b) with a height,
wherein the width of the bottom (154a) is proportional to the height of the side walls (154b).

3. Optical vital signs sensor (100) according to claim 2, wherein
the bottom (154a) is flat and the side walls (154b) are perpendicular with respect to the bottom (154a).

4. Optical vital signs sensor (100) according to claim 2, wherein a transition between the bottom and at one of the side walls of the gap (154) and/or a transition between at least one of the side walls and one of the first and second protrusions (152, 153) is rounded and/or chamfered.

5. Optical vital signs sensor (100) according to claim 1, wherein
the contact surface (101) comprises an elevated portion (102),
wherein the transparent portion (150) is arranged at the elevated portion (102).

6. Optical vital signs sensor according to claim 1, further comprising
a printed circuit board (130) having a first plane (131),
wherein the at least one light source (110) is arranged at a first angle (α1) with respect to the plane (131) of the printed circuit board (130),
wherein the at least one photo detector unit (120) is arranged at a second angle (α2) with respect to the plane (131) of the printed circuit board (130).

7. Optical vital signs sensor according to claim 6, wherein
the gap (154) comprises side walls (154b) which are arranged at the first or second angle (α1, α2) with respect to the plane (131) of the printed circuit board (130).

8. Optical vital signs sensor according to claim 1,
wherein the transparent portion (150) comprises a transparent glass layer or a transparent plastic layer.

9. Optical vital signs sensor according to claim 1, wherein the transparent portion (150) is transparent for at least a wavelength of the at least one light source (110).

10. Optical vital signs sensor according to claim 1,
wherein the transparent portion (150) is only transparent at the first and second protrusion (152, 153).

11. Optical vital signs sensor according to claim 10, wherein
the transparent portion (150) is only transparent at side walls (154b) of the gap (154),
wherein the side walls (154b) are inclined with respect to a bottom (154a) of the gap (154).

12. Method of producing an optical vital signs sensor (100) which is configured to measure or determine vital signs of a user, wherein said optical vital signs sensor is photoplethysmographic sensor (PPG), comprising the steps of
providing at least one light source (110) configured to generate light which is directed towards a skin of a user,
providing at least one photo detector unit (120) configured to detect light, wherein said light being indicative of a reflection and/or transmission of light emitted in or from the skin (1000) of a user,
providing a contact surface which can be pressed against the skin (1000) of a user, and
providing a transparent portion (150) with at least one first protrusion (152) arranged over the at least one light unit (110), at least one second protrusion (153) arranged over the at least one photo detector unit (120) and a gap (154) arranged between the first and second protrusion (152, 153).
